Europäisches Patentamt

European Patent Office

Office européen des brevets

(11) Publication number: 0 375 260
A1

## (12) EUROPEAN PATENT APPLICATION

(21) Application number: 89313018.7

(22) Date of filing: 13.12.89

(51) Int. Cl.5: C12P 21/02, C07K 1/02, C07K 7/10

(30) Priority: 15.12.88 GB 8829432

(43) Date of publication of application:
27.06.90 Bulletin 90/26

(84) Designated Contracting States:
AT BE CH DE ES FR GB GR IT LI LU NL SE

(71) Applicant: CELLTECH LIMITED
216 Bath Road
Slough Berkshire SL1 4EN(GB)

(72) Inventor: Carne, Alexander Fraser
3 Ye Meads Cottages Marsh Lane
Taplow Bucks. SL6 ODH(GB)
Inventor: Rose, Keith
7 Chemin de la Boisserette
CH-1208 Geneva(CH)
Inventor: Offord, Robin Ewart
65 Route d'Ornex
CH-1249 Collex Bossy(CH)

(74) Representative: Hallybone, Huw George et al
CARPMAELS AND RANSFORD 43
Bloomsbury Square
London WC1A 2RA(GB)

(54) A process for the production of calcitonin gene related peptides.

(57) Calcitonin gene related peptides are produced by enzyme-assisted reverse proteolysis. Reaction of a C-terminal truncated calcitonin gene related peptide or a protected derivative with a C-terminal amidated peptide or amino acid, or ammonia in the presence of a hydrolase, yields a calcitonin gene related peptide (CGRP) in good yield and high purity. The CGRP product may be a naturally occurring CGRP or an analogue thereof, such as human $\alpha$ - or $\beta$ -CGRP. Particular hydrolases include serine proteases such as chymotrypsin, trypsin, thrombin, plasmin, elastase, endoproteinase Lys C; endoproteinase Glu C, or endoproteinase Arg C, acid proteases such as pepsin; metalloproteinases such as thermolysin; and sulphydryl proteinases such as papain or clostripain.

## A PROCESS FOR THE PRODUCTION OF CALCITONIN GENE RELATED PEPTIDES

Field of the Invention

This invention relates to a process for the enzymatic production of calcitonin gene related peptides. More particularly, the invention relates to a process for producing calcitonin gene related peptides by enzyme-assisted reverse proteolysis.

Background to the Invention

Calcitonin gene related peptides (CGRPs) have been identified in a number of species [see, for example, Amara et al., (1982) Nature 298, 240-244 and UK Patent Specification No. 2141430]. They constitute a family of closely related peptide hormones with a number of physiological actions, on inter alia the vascular and neural systems. The hormones, and chemically synthesised derivatives (see for example European Patent Specification No. 212432), have been proposed for use as medicaments, for example for use in the treatment of hypertension, cardiac insufficiency or other cardiac circulatory disorders.

Existing processes for the production of CGRPs fall into two general categories. Thus, the peptides may be chemically synthesised, (for example, by the sequential coupling of appropriate amino acids) or, alternatively, they may be produced in yeast or bacteria using recombinant DNA technology.

In general, although chemical techniques are acceptable for generating CGRPs on a small scale, they are not very attractive commercially, primarily since they are expensive to operate. Production of CGRPs by a yeast or bacteria containing a recombinant CGRP gene offers advantages over chemical methods in that the recombinant gene approach is generally cheaper, is more amenable to scale-up, and produces purer products.

A major problem with the recombinant gene approach however is that it is not possible to direct yeast or bacteria to produce CGRPs with the C-terminal α-amide groups which appear necessary for their activity. An additional and expensive processing step must therefore be undertaken, once the peptide has been isolated, to introduce the required amide function. One previously suggested solution to this, is to direct the organism to produce a CGRP with an additional C-terminal glycine residue, and in a subsequent step to convert the glycine residue to the required amide group by use of an amidating enzyme (see European Patent Specification No. 188400).

We have now found a new enzymatic process for the production of CGRPs, which essentially utilises a hydrolytic enzyme to catalyse the coupling of an amidated peptide or amino acid, or ammonia to a C-terminal carboxylic acid or ester of a peptide. The process is particularly suitable for use in conjunction with recombinant DNA technology, as part of a microbiologically based synthesis of CGRPs. It advantageously produces CGRPs in good yields and high purities and can be operated on a commercial scale at low cost.

Summary of the Invention

Thus according to one aspect of the invention we provide a process for the production of a calcitonin gene related peptide which comprises reacting a C-terminal truncated calcitonin gene related peptide or a protected derivative thereof with a C-terminal amidated peptide or amino acid, or ammonia in the presence of a hydrolase until said calcitonin gene related peptide or protected derivative thereof accumulates, recovering the accumulated calcitonin gene related peptide or protected derivative, and, where necessary, deprotecting the protected derivative.

The calcitonin gene related peptide (hereinafter referred to as CGRP) product produced according to the invention may have the amino acid sequence of a naturally occurring CGRP, or an analogue thereof. Thus for example the CGRP product may have the amino acid sequence of an animal, e.g. mammalian, such as human or rat, or chicken, α-or β-CGRP. Alternatively, the CGRP product may be an analogue of a naturally occurring CGRP, for example a naturally occurring CGRP wherein at least one amino acid has been replaced by a different amino acid; a naturally occurring CGRP containing one or more additional amino acids; or a naturally occurring CGRP wherein at least one amino acid is absent.

Protected derivatives of the CGRP product include those derivatives wherein the N-terminal amino group and/or one or more side-chain groups is in a protected form. Particular examples include protected CGRP derivatives wherein the N-terminal amino group is substituted by an acyl group such as alkanoyl (e.g. acetyl), aroyl (e.g. benzoyl), alkoxycarbonyl (e.g. t-butoxycarbonyl), or aralkoxycarbonyl (e.g. benzyloxycarbonyl) group or an aralkyl (e.g. benzyl) group; and/or wherein side-chain groups such as carboxyl or hydroxyl are protected by esterification or etherification, as appropriate, and cysteine groups are protected by acylation or alkylation.

The C-terminal truncated CGRP (hereinafter referred to as t-CGRP), or protected t-CGRP derivative, for use as a starting material in the process according to the invention, in general may be a CGRP or protected derivative as defined above wherein either the C-terminal amino acid amide or a C-terminal peptide containing the C-terminal amino acid amide is absent, or where the C-terminal carboxamide function has been replaced by a carboxylic acid or ester group. The t-CGRP or protected derivative may have as its C-terminus a carboxylic acid ($-CO_2H$) or ester $-CO_2R$, (where R is for example a group selected from alkyl, e.g. methyl, aryl, e.g. phenyl, or aralkyl, e.g. benzyl) group.

Particular examples of t-CGRP's for use in the invention include des-Phe$^{37}$NH$_2$-CGRP, des-Ala$^{36}$-Phe$^{37}$NH$_2$-CGRP (where CGRP is as defined previously) Phe$^{37}$CO$_2$H-CGRP or protected derivatives thereof.

The C-terminal amidated peptide or amino acid starting material will in general be of formula H[Y]-$_n$NH$_2$ [wherein n is zero or an integer 1, 2, 3, or more, and Y represents the residue of an amino acids [for example a group of formula $-HN-CH(R^1)-CO-$, where $R^1$ is a hydrogen atom or an organic group e.g. a group of the type attached to the $\alpha$-carbon atom in naturally occurring amino acids] with the proviso that when two or more Y groups are present they may be the same or different]. Particular examples include -NH$_3$, Ala-Phe-NH$_2$ or Phe-NH$_2$.

In general the amino acids described above (either singly or as part of a peptide structure) are L-isomers.

The hydrolase for use in the process according to the invention may be any hydrolytic enzyme that is capable of catalysing the formation of a peptide bond between the C-terminal carboxyl group of the t-CGRP and the N-terminal amino group of the amidated peptide or amino acid starting materials. The hydrolase may be of animal, e.g. mammalian; fungal; or bacterial origin, and may be an endopeptidase or an exopeptidase. Particular examples include serine proteases such as chymotrypsin, trypsin, thrombin, plasmin, elastase endoproteinase Lys C, endoproteinase Glu C, or endoproteinase Arg C; acid proteases such as pepsin; metalloproteinases, for example calcium dependent metalloproteinases such as thermolysin; and sulphydryl proteinases such as papain or clostripain.

Particularly useful hydrolases include metalloproteinases, especially thermolysin.

If desired, the hydrolase may be in an immobilised form, for example coupled to or adsorbed on an insoluble support such as a cellulose, polyamide or silica, or a cross-linked dextran or agarose, or entrapped in a suitable matrix such as a polyacrylamide or alginate fibre, using conventional procedures (see, for example, Methods in Enzymology 44, ed. K. Mosbach, Academic Press, New York, 1976).

The process according to the invention is particularly useful for the production of a human CGRP and, in a preferred aspect, we provide a process for the production of a human CGRP which comprises reacting a C-terminal truncated human CGRP, or a protected derivative thereof, with a C terminal amidated peptide, amino acid or ammonia in the presence of a hydrolase until said CGRP or protected derivative accumulates, recovering the accumulated CGRP or protected derivative and, where necessary, deprotecting the protected derivative.

In this aspect of the invention, the CGRP Product may be, for example, human $\beta$-CGRP or, especially, human $\alpha$-CGRP, or an analogue or protected derivative thereof.

Preferably, the starting materials for use in this aspect of the invention are des-Phe$^{37}$NH$_2$-CGRP (where CGRP represents the first thirty-six amino acids of a human CGRP) and Phe-NH$_2$, and the hydrolase is thermolysin.

In the process according to the invention, the exact conditions used will depend on the nature of the enzyme and starting materials and may need to be determined empirically beforehand, on a small-scale, in accordance with conventional practice. In general, conditions will be chosen [for example by adjusting variables such as the reaction solvent, pH and enzyme concentration as discussed below] to avoid as far as possible the occurrence of unwanted side-reactions, such as enzyme catalysed hydrolysis of the starting materials.

The process according to the invention may be performed in a suitable solvent, for example an aqueous medium containing a water miscible organic solvent. Suitable organic solvents include for example glycols, such as butane-1,4-diol, substituted amides, such as dimethylformamide or N,N-dimethylacetamide, dimethylsulphoxide, ethers, such as as tetrahydrofuran, and alkanols, such as methanol or ethanol.

The pH of the reaction solution will depend on the enzyme selected for use, but in general will be in the range pH3.5 to pH10.5 [It will be appreciated that with the reaction solvents in use, a reference to pH in this context is a reference to an apparent pH]. The temperature of the reaction solution may be varied in the range 0°C to 80°C e.g. 15°C to 45°C.

The t-CGRP and C-terminal amidated peptide or amino acid starting materials are either known, readily available, compounds, or may be prepared by chemical or recombinant DNA techniques using known methods (see for example European Patent Specification No. 188400). The hydrolase may be obtained from conventional sources.

In the process according to the invention the starting materials and hydrolase may generally be added to the reaction solvent in any order, but preferably the enzyme is added last. The starting materials may be L-isomers, or, if desired mixtures of D- and L-isomers. It may be necessary to dissolve one or both starting materials in a minimum volume of water, or other suitable solvent, for example as described above, before addition to the reaction solvent. The exact quantities of starting materials and hydrolase used may be varied within a wide range, depending on the nature of the substrates and enzyme, and may, for example, range from an enzyme-substrate ratio of around 1:5 to around 1:50, for example around 1:10 to around 1:25. Once the reaction components have been mixed in the solvent, the reaction is allowed to proceed until sufficient of the desired product has accumulated. The appearance of the product during the process may be monitored using conventional techniques, for example by reversed phase high performance liquid chromatography.

Once sufficient of the desired product has accumulated it may be recovered using conventional techniques for the recovery of polypeptides from mixtures, for example by chromatography, e.g. by high performance liquid chromatography, affinity chromatography or ion exchange chromatography.

Where the product is a protected CGRP it may be deprotected using standard deprotection methods, such as by acid hydrolysis, to yield the desired CGRP product.

Description of Specific Embodiments

The following Examples illustrate the invention.

In the Examples the 'pH' is that value indicated at a glass electrode (Russell Standard combination electrode, model CMAWL) calibrated at pH4.0 and 7.0 with Radiometer aqueous standards, and no other corrections were made. All temperatures are in °C. In all Examples stirring or sonication was necessary to facilitate dissolution of the nucleotide.

Example 1

Thermolysin-Catalysed Preparation of human α-CGRP using Phe-NH₂ and des - Phe-NH₂-Human - α-CGRP in Butane-1,4-Diol

90% Butane-1,4-diol was prepared by adding 1 volume of water to 9 volumes of butane-1,4-diol with thorough mixing. L-Phe-NH₂ (Sigma;328.4mg) was dissolved in the diol solution (2.0ml). The pH of the amino component was adjusted to pH6.0 by the addition of glacial acetic acid (0.25ml). Desphe-NH₂-human - α-CGRP (1.0mg) was dissolved in water (0.04ml) and the amino component (0.2ml) added slowly with continuous stirring.

A solution of thermolysin was prepared at a concentration of 50mg/ml in 0.1M calcium acetate. 0.002ml of this solution was added to the reaction mixture, making sure that the enzyme was fully dispersed. The mixture was left to react for 20 hours at 22°. An equal volume of acetic acid was added to stop the reaction and the mixture then diluted with 2 volumes of 0.1% trifluoroacetic acid. The mixture was then analysed by reverse-phase HPLC carried out under the following conditions:
Column: Nucleosil 5mM, 300A, C₈ (Machery Nagel, Duren, West Germany), 250 x 4mm, linear gradient 10 - 60% B, 50min.
A: 1.0g trifluoroacetic acid in water (HPLC grade),
B: 1.0g trifluoroacetic acid, 100ml water, 900ml of acetonitrile.

Peptide material was determined by UV absorbance at 215nm.

Material corresponding to authentic human α-CGRP was collected in 70% yield and its identity with human α-CGRP confirmed by FAB-MS (Mass peak : 3789.2).

Example 2

Thermolysin-Catalysed Preparation of human α-CGRP using Phe-NH₂-Human α-CGRP in Dimethylsulphoxide

The process according to Example 1 was repeated except 50% aqueous dimethylsulphoxide was used instead of butane-1,4-diol, and the reaction time was decreased to 4 hours at 33°. Human α-CGRP, as analysed by reverse-phase HPLC and FAB-MS, (for conditions see Example 1), was obtained in 30% yield.

Example 3

Endopeptidase-Catalysed Preparation of human α-CGRP using Ala-Phe-NH₂ and des - Ala-PheNH₂-human - α-CGRP in Butane-1,4-Diol

L-Ala-Phe-NH₂.HCl (271.6mg) was dissolved in 1.0ml of 90% butane-1,4-diol (see Example 1). The pH was adjusted to 5.5 by the careful addition of solid TRIS.

des Ala-PheNH₂-human-α-CGRP (1.0mg) was dissolved in 0.1ml of the amino component.

0.45mg of Lysyl endopeptidase (Achromobacter Protease I) was dissolved in water (0.045ml) and 0.004ml added to the reaction mixture. The solution was thoroughly mixed and left at 22° for 4 hours. The reaction was then stopped by the addition of an equal volume of acetic acid and diluted with a further 2 volumes of 0.1% trifluoroacetic acid. Human α-CGRP, as analysed by HPLC and FAB-MS (for conditions see Example 1), was obtained in 70% yield.

Example 4

Endopeptidase-Catalysed Preparation of Human α-CGRP using Ala-Phe-NH₂ and des - Ala-PheNH₂-CGRP in N,N-Dimethylacetamide

L-Ala-PheNH₂ (271.6mg) was dissolved in N,N-dimethylacetamide (80%; 2.0ml), and the pH adjusted to 5.5 by the addition of solid TRIS. The reaction was then carried out as for Example 3 to give α-CGRP (collected by HPLC and characterised by FAB-MS - see Example 1) in 50% yield.

**Claims**

1. A process for the production of a calcitonin gene related peptide which comprises reacting a C-terminal truncated calcitonin gene related peptide or a protected derivative thereof with C-terminal amidated peptide or amino acid, or ammonia in the presence of a hydrolase until said calcitonin gene related peptide or protected derivative thereof accumulates, recovering the accumulated calcitonin gene related peptide or protected derivative, and, where necessary, deprotecting the protected derivative.

2. A process as claimed in Claim 1 wherein the hydrolase is a serine protease, an acid protease, a metalloproteinase or a sulphydryl proteinase.

3. A process as claimed in Claim 2 wherein the hydrolase is a metalloproteinase.

4. A process as claimed in Claim 3 wherein the hydrolase is thermolysin.

5. A process as claimed in any of the preceding claims wherein the calcitonin gene related peptide has the amino acid sequence of a naturally occurring calcitonin gene related peptide or an analogue thereof.

6. A process as claimed in Claim 5 wherein the calcitonin gene related peptide is a mammalian calcitonin gene related peptide or an analogue thereof.

7. A process as claimed in Claim 6 wherein the calcitonin gene related peptide is a human α- or β-calcitonin gene related peptide or an analogue thereof.

8. A process as claimed in Claim 7 wherein the calcitonin gene related peptide is human α-calcitonin gene related peptide.

9. A process for the production of human α-calcitonin gene related peptide which comprises reacting des-Phe³⁷NH₂-human α-calcitonin gene related peptide or a protected derivative thereof with Phe-NH₂ in the presence of thermolysin until said human α-calcitonin gene related peptide or protected derivative thereof accumulates, recovering the accumulated human α-calcitonin gene related peptide or protected derivative, and, where necessary, deprotecting the protected derivative.

10. A process as claimed in any of the preceding claims wherein the starting materials and hydrolase are reacted in an aqueous medium containing a water miscible organic solvent.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 270 376 (TOYO JOZO K.K.) --- | | C 12 P 21/02 |
| A | US-A-4 086 136 (J. ISOWA et al.) --- | | C 07 K 1/02 |
| D,A | EP-A-0 188 400 (CIBA-GEIGY A.G.) ----- | | C 07 K 7/10 |

TECHNICAL FIELDS
SEARCHED (Int. Cl.5)

C 12 P
C 07 K
C 12 N

The present search report has been drawn up for all claims

| Place of search | Date of completion of the search | Examiner |
|---|---|---|
| THE HAGUE | 19-03-1990 | NOVOA Y SANJURJO M.A. |